# FASCICULE DE BREVET EUROPEEN

(11) **EP 3 927 403 B1**
(45) Date de publication et mention de la délivrance du brevet: **05.04.2023**
(21) Numéro de dépôt: 20710237.7
(22) Date de dépôt: 18.02.2020
(51) Int. Cl.: A61M 11/00, A61M 15/08, A61M 15/00, A61M 5/24, B05B 11/00, A61M 5/30, B05B 11/02

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 22.02.2019 FR 1901825
(43) Date de publication de la demande: 29.12.2021
(73) Titulaire: APTAR France SAS, 27110 Le Neubourg (FR)
(72) Inventeur: LEBEL, Baptiste, 27100 Val de Reuil (FR)
(74) Mandataire: CAPRI
(86) Numéro de dépôt international: PCT/FR2020/050292
(87) Numéro de publication internationale: WO 2020/169915

(56) Documents cités:
- WO-A1-2015/024653
- WO-A1-2019/008264
- FR-A1- 2 847 834

## Description

La présente invention concerne un dispositif de distribution de produit fluide, notamment sous forme de spray nasal.

Les dispositifs de distribution de produit fluide sont bien connus dans l'état de la technique.

Généralement, ces dispositifs comportent un réservoir contenant plusieurs doses de fluides à distribuer, sur lequel est monté un organe de distribution, tel qu'une pompe doseuse, pour distribuer une dose à chaque actionnement. Un inconvénient des pompes doseuses concerne l'amorçage, qui impose un ou plusieurs actionnements préalables avant de garantir la distribution d'une dose complète. Ce problème ne se pose pas seulement avant le premier actionnement, mais il peut se poser avant chaque actionnement, surtout s'il se passe du temps, typiquement plusieurs jours, entre deux actionnements successifs. Or, avec certains produits fluides, tels que des produits pharmaceutiques dangereux ou particulièrement onéreux, typiquement pour des traitements anti-migraines, de tels actionnements d'amorçage ne sont pas souhaitables, car ils distribuent nécessairement un peu de fluide, avec donc des risques de mauvais dosage pour l'utilisateur et/ou du gaspillage d'un produit actif qui coûte cher. Par exemple dans un traitement anti-migraine impliquant la distribution successive de quatre doses de médicament, il faut pratiquement remplir le réservoir avec cinq voire six doses de produit fluide pour garantir qu'il y aura bien quatre doses complètes distribuées. Le surcoût en produit actif est donc très important.

Il existe également des dispositifs comportant un réservoir, contenant le produit fluide à distribuer, et un piston, qui est monté coulissant dans ledit réservoir, et qui est déplacé pour distribuer sélectivement le produit fluide contenu dans ledit réservoir. Lorsque le réservoir contient plusieurs doses de produit fluide à distribuer lors de plusieurs actionnements successifs, le déplacement du piston se fait en plusieurs courses d'actionnement successives, de sorte qu'une première dose est distribuée lors d'un premier actionnement, une seconde dose est distribuée lors d'un deuxième actionnement, etc. Avec ce type de dispositif multidose, il se pose le problème de l'altération du produit fluide restant dans le réservoir après la première utilisation. En effet, lors du premier actionnement, le réservoir est ouvert, de sorte que son contenu est susceptible d'être contaminé, notamment lorsque le dispositif est stocké entre deux actionnements successifs. Or, selon le type de produit fluide qui est distribué par le dispositif, notamment lorsqu'il s'agit d'un médicament, il peut être important d'éviter tout risque de contamination.

Pour surmonter cet inconvénient, le document WO2019008264 propose d'isoler le produit fluide entre deux actionnements. Ce dispositif peut toutefois présenter des inconvénients. Ainsi, l'actionnement peut nécessiter plusieurs actions distinctes de l'utilisateur, ce qui peut s'avérer compliqué dans certaines situations. De plus, l'encombrement axial peut être relativement important. La précision de dosage peut être dépendante de la force exercée par l'utilisateur lors de l'actionnement.

Les documents FR2847834, WO2015024653, US2005029288, US2007000950 et US2008210229 décrivent d'autres dispositifs de l'état de la technique.

La présente invention a pour but de fournir un dispositif de distribution de produit fluide qui ne reproduit pas les inconvénients susmentionnés.

La présente invention a ainsi pour but de fournir un dispositif de distribution de produit fluide dont l'actionnement est sûr et fiable à chaque actionnement.

La présente invention a aussi pour but de fournir un dispositif de distribution de produit fluide dont l'encombrement axial est réduit.

La présente invention a aussi pour but de fournir un dispositif de distribution de produit fluide dont la précision de dosage est améliorée à chaque actionnement.

La présente invention a aussi pour but de fournir un dispositif de distribution de produit fluide qui évite tout gaspillage de produit fluide avant et/ou après chaque actionnement.

La présente invention a aussi pour but de fournir un dispositif de distribution de produit fluide qui assure une protection du produit fluide entre deux actionnements.

La présente invention a également pour but de fournir un tel dispositif de distribution de produit fluide qui soit simple et peu coûteux à fabriquer et à assembler.

La présente invention a donc pour objet un dispositif de distribution de produit fluide comportant un corps pourvu d'un orifice de distribution et d'un élément de perçage, ledit corps contenant un réservoir contenant au moins deux doses de produit fluide, ledit réservoir comportant une ouverture axiale proximale obturée par une membrane et une ouverture axiale distale obturée par un piston, monté coulissant dans ledit réservoir pour distribuer une dose de produit fluide à chaque actionnement, ledit réservoir étant axialement déplaçable par rapport audit corps entre une position de repos, dans laquelle ledit élément de perçage ne traverse pas ladite membrane, et une position de distribution, dans laquelle ledit élément de perçage traverse ladite membrane, ladite membrane étant adaptée à se refermer de manière étanche après chaque actionnement, lorsque ledit élément de perçage ressort dudit réservoir, ledit dispositif comportant un système d'actionnement latéral comprenant un organe d'actionnement, déplaçable latéralement par rapport audit corps entre une position de repos et une position d'actionnement, ledit organe d'actionnement étant élastiquement sollicité vers sa position de repos, ledit organe d'actionnement comportant une première came coopérant avec ledit réservoir et une seconde came coopérant avec ledit piston, de telle sorte que le déplacement dudit organe d'actionnement vers sa position d'actionnement déplace d'abord ledit réservoir vers sa position de distribution puis fait coulisser ledit piston dans ledit réservoir, pour distribuer une dose de produit fluide.

Avantageusement, ledit organe d'actionnement est monté pivotant sur une partie de corps inférieur, fixée audit corps.

Avantageusement, ledit organe d'actionnement est élastiquement sollicité vers sa position de repos par un ressort de rappel.

Avantageusement, ladite première came et ladite seconde came sont formées par des rainures ou fentes réalisées dans ledit organe d'actionnement.

Avantageusement, ladite première came coopère avec au moins un ergot solidaire dudit réservoir, ledit ergot coulissant dans ladite première came lors de l'actionnement, et ladite seconde came coopère avec au moins un ergot solidaire dudit piston, ledit ergot coulissant dans ladite seconde came lors de l'actionnement.

Avantageusement, ladite première came comporte une première partie oblique axialement et une seconde partie sensiblement horizontale, ladite première partie coopérant avec ledit ergot au début de l'actionnement pour le pousser axialement vers le haut, ce qui déplace axialement ledit réservoir vers le haut, provoquant le perçage de ladite membrane par ledit élément de perçage, et ladite seconde partie coopère avec ledit ergot en fin d'actionnement, de sorte qu'après perçage de ladite membrane, ledit réservoir n'est pas davantage poussé vers le haut.

Avantageusement, ledit ergot est formé sur un organe de support fixé audit réservoir.

Avantageusement, ladite seconde came comporte une première partie et une seconde partie oblique axialement, ladite première partie coopérant avec ledit ergot au début de l'actionnement de sorte que ledit ergot ne subit aucune force axiale par ladite came et ne bouge pas au début dudit actionnement, et ladite seconde partie coopérant avec ledit ergot en fin d'actionnement pour pousser axialement vers le haut ledit ergot, ce qui déplace axialement vers le haut ledit piston dans ledit réservoir pour distribuer une dose du produit fluide contenu dans ledit réservoir.

Avantageusement, ladite première partie est plus large que ledit ergot.

Avantageusement, ledit ergot est formé sur un manchon d'actionnement coopérant avec une tige de piston fixée audit piston.

Avantageusement, ladite tige de piston comporte une denture coopérant avec au moins un crochet dudit manchon d'actionnement.

Avantageusement, un élément d'indication est fixé à ladite tige de piston, ledit élément d'indication étant visible dans une fenêtre de visualisation, avantageusement pourvue d'indices d'indication tels que des graduations et/ou des indices alphanumériques et/ou colorés.

Avantageusement, ledit organe d'actionnement comporte une projection déformable coopérant en début d'actionnement avec une butée du corps pour générer une accumulation d'énergie dans la main de l'utilisateur.

Avantageusement, ledit élément de perçage est solidaire d'un insert fixé dans ledit corps, en amont dudit orifice de distribution.

Avantageusement, un profil de pulvérisation est prévu directement en amont dudit orifice de distribution.

Ces avantages et caractéristiques et d'autres de la présente invention apparaitront plus clairement au cours de la description détaillée suivante, faite en référence aux dessins joints, donnés à titre d'exemples non limitatifs, et sur lesquels:
La figure 1 est une vue schématique partielle de côté d'un dispositif de distribution de produit fluide selon un mode de réalisation avantageux de la présente invention, en position de repos,
La figure 2 est une vue en section transversale du dispositif de la figure 1,
La figure 3 est une vue similaire à celle de la figure 1, en cours d'actionnement, après perçage du réservoir et avant distribution d'une dose,
La figure 4 est une vue en section transversale du dispositif de la figure 3,
La figure 5 est une vue similaire à celle de la figure 3, en cours d'actionnement, pendant la distribution d'une dose,
La figure 6 est une vue en section transversale du dispositif de la figure 5,
La figure 7 est une vue similaire à celle de la figure 6, en cours d'actionnement, après la distribution d'une dose et avant la fermeture du réservoir,
La figure 8 est une vue similaire à celle de la figure 7, après actionnement, avec le dispositif prêt pour l'actionnement suivant, et
Les figures 9 à 12 sont des vues schématiques illustrant l'indicateur de dose selon un mode de réalisation avantageux.

La présente invention sera décrite ci-après en référence à un exemple de réalisation schématique. Les formes et dimensions des différentes parties constitutives ne sont qu'illustratives et non limitatives. Il est entendu que la présente invention s'applique de manière plus générale à tout type de dispositif contenant au moins deux doses.

Les termes "proximal" et "distal" se réfèrent à l'orifice de distribution. Le terme "axial" se réfère à l'axe central longitudinal du réservoir. Les termes "haut", "bas" et "horizontal" se réfèrent à la position droite du dispositif représentée sur les figures.

En se référant aux figures, le dispositif de distribution comporte un corps 1 pourvu d'un orifice de distribution 2. Eventuellement, comme dans l'exemple représenté, l'orifice de distribution 2 est réalisé dans une tête de distribution 9 fixée sur le corps 1. Un capot amovible 8 peut être prévu pour protéger ledit orifice de distribution 2 lorsque le dispositif n'est pas utilisé.

Ledit corps 1 contient un réservoir 10 contenant plusieurs doses de produit fluide, notamment un médicament liquide, par exemple à pulvériser dans le nez d'un utilisateur. Ce réservoir 10 comporte avantageusement un corps creux 11 principalement cylindrique comportant une ouverture axiale proximale et d'une ouverture axiale distale. L'ouverture distale est obturée de manière étanche par un piston 20, monté coulissant dans ledit réservoir 10. L'ouverture proximale est obturée par une membrane 30, de préférence étanche. Les dessins montrent une ouverture proximale formée sur une partie cylindrique de diamètre réduit, mais ceci n'est pas obligatoire, et le corps creux 11 formant le réservoir 10 pourrait aussi être parfaitement cylindrique.

Ledit piston 20 coopère avec une tige de piston 40 qui s'étend axialement hors dudit réservoir 10. Ladite tige de piston 40 coopère donc d'une part avec ledit piston 20, et elle coopère d'autre part avec un système d'actionnement latéral, comme cela sera décrit plus amplement ci-après.

La tige de piston 40 est crantée, c'est-à-dire qu'elle comporte une denture 45 s'étendant longitudinalement le long de ladite tige de piston. Un manchon d'actionnement 70 est monté autour de ladite tige de piston 40, ledit manchon d'actionnement 70 comportant au moins un crochet 71 coopérant avec ladite denture 45 de ladite tige de piston. De préférence, comme visible sur les figures, ce crochet 71 est formé au niveau de l'extrémité axiale proximale dudit manchon d'actionnement 70. Le manchon d'actionnement 70 comporte en outre au moins un ergot 72 dont la fonction sera plus amplement décrite ci-après.

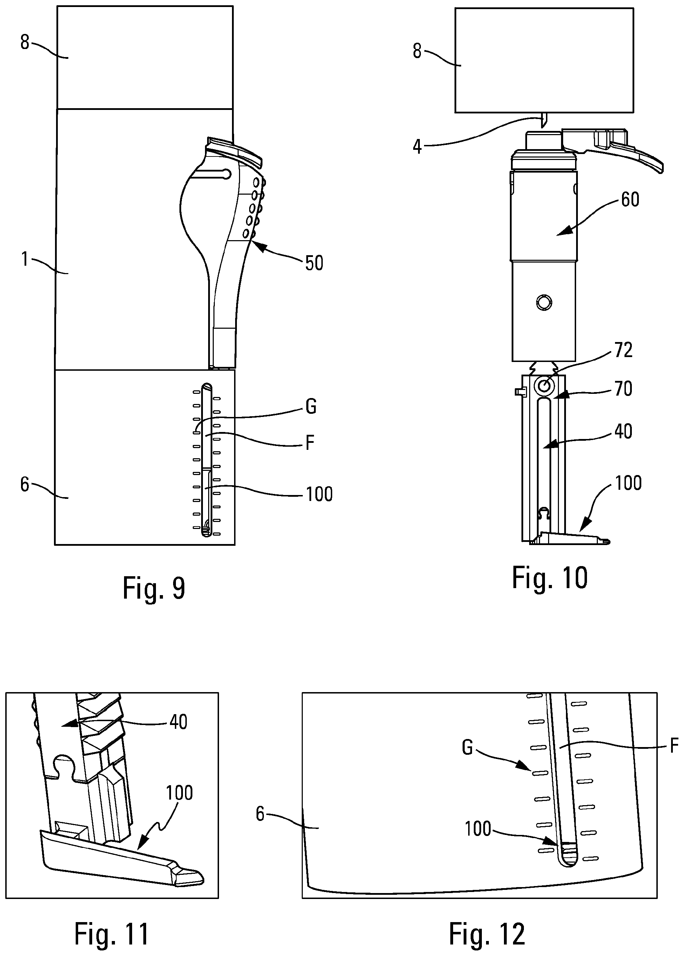

Un indicateur de dose avantageux est représenté sur les figures 9 à 12. Cet indicateur comporte avantageusement un élément d'indication 100 fixé à la tige de piston 40, de préférence à son extrémité axiale distale, et se déplaçant donc axialement ensemble avec celle-ci. Une fenêtre de visualisation F réalisée dans une partie de corps inférieur 6 fixée au corps 1 permet de visualiser le déplacement dudit élément d'indication 100 à chaque actionnement. De préférence, ladite fenêtre F est pourvue d'indices d'indication, tels que par exemple des graduations G. En variante, elle pourrait aussi comporter des indices alphanumériques et/ou colorés. D'autres variantes sont aussi envisageables.

Le corps 1 et/ou la tête de distribution 9 peut comporter, en amont dudit orifice de distribution 2, un insert 3 supportant un élément de perçage creux 4, tel qu'une aiguille ou une canule, destinée à percer ladite membrane 30 du réservoir 10.

Ledit insert 3 peut également définir un profil de pulvérisation 5 directement en amont dudit orifice de distribution 2, pour générer un spray, notamment un spray nasal. Ce profil de pulvérisation 5 peut être formé de manière classique dans le bord axial proximal dudit insert 3 et/ou dans la paroi de fond dudit corps 1 et/ou de ladite tête de distribution 9, entourant ledit orifice de distribution 2 et faisant face audit bord axial proximal de l'insert 3. Ce profil de pulvérisation 5 peut comporter des canaux de tourbillonnement et une chambre de tourbillonnement.

Le réservoir 10 est monté axialement coulissant dans ledit corps 1 entre une position de repos, dans laquelle ledit élément de perçage 4 ne traverse pas ladite membrane 30, et une position de distribution, dans laquelle ledit élément de perçage 4 traverse ladite membrane 30. Le réservoir 10 peut être fixé dans un organe de support 60, qui peut notamment envelopper ledit réservoir 10 comme représenté dans l'exemple des figures. L'organe de support 60 comporte en outre au moins un ergot 61 dont la fonction sera plus amplement décrite ci-après. En variante, ledit au moins un ergot 61 pourrait être formé directement sur le réservoir 10.

Pour réaliser les actionnements successifs de dispositif, il est prévu un système d'actionnement latéral.

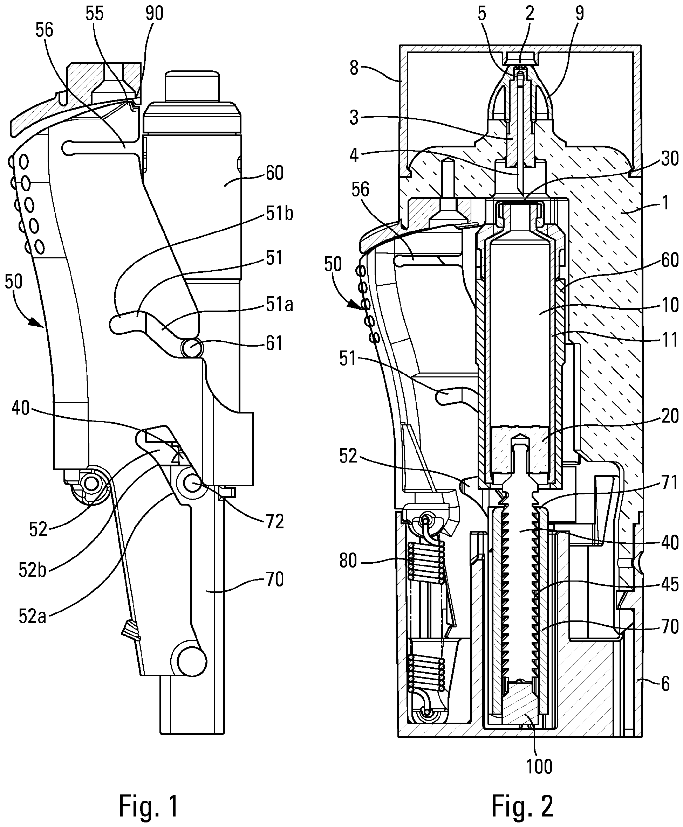

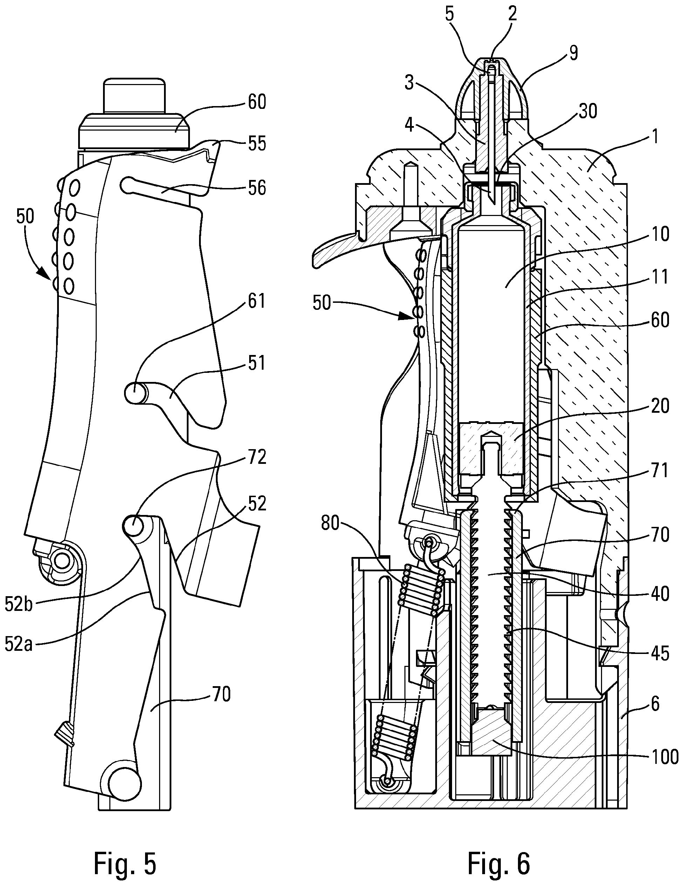

Ce système d'actionnement latéral comporte un organe d'actionnement 50, déplaçable latéralement par rapport audit corps 1 entre une position de repos, visible sur les figures 1 et 2, et une position d'actionnement, visible sur les figures 5 et 6. Dans l'exemple des figures, cet organe d'actionnement 50 est monté pivotant sur une partie de corps inférieur 6, fixée audit corps 1.

Cet organe d'actionnement 50 comporte une première came 51 et une seconde came 52, de préférence formées par des rainures ou fentes.

La première came 51 coopère avec ledit ergot 61 qui coulisse dans la rainure 51 lors de l'actionnement. La seconde came 52 coopère avec ledit ergot 72 qui coulisse dans la rainure 52 lors de l'actionnement.

Avantageusement, la rainure 51 comporte une première partie 51a et une seconde partie 51 b. La première partie 51a coopère avec ledit ergot 61 au début de l'actionnement, et la seconde partie 51b coopère avec ledit ergot 61 en fin d'actionnement.

De préférence, la première partie 51a est oblique axialement, de sorte que lorsque l'utilisateur fait pivoter l'organe d'actionnement 50 de sa position de repos vers sa position d'actionnement, l'ergot 61 est poussé axialement vers le haut en coulissant dans ladite première partie 51a, ce qui déplace axialement ledit réservoir 10 vers le haut, provoquant le perçage de la membrane 30 par l'élément de perçage 4, comme visible sur les figures 1 à 4. Avantageusement, la seconde partie 51b est sensiblement horizontale, de sorte qu'après perçage de la membrane 30, le réservoir 10 n'est pas davantage poussé vers le haut.

De préférence, la seconde rainure 52 comporte une première partie 52a et une seconde partie 52b. La première partie 52a coopère avec ledit ergot 72 au début de l'actionnement, et la seconde partie 52b coopère avec ledit ergot 72 en fin d'actionnement.

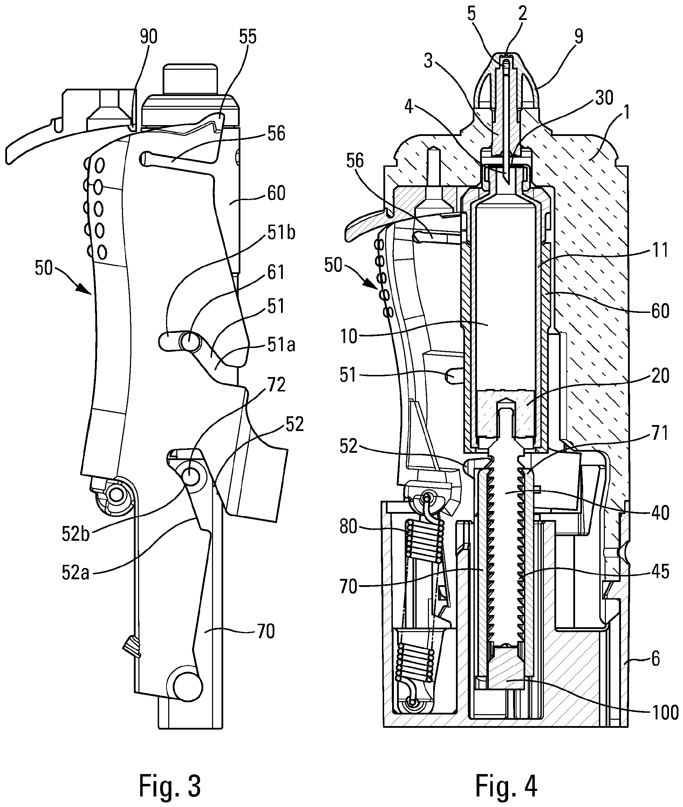

Avantageusement, lorsqu'il se déplace dans la première partie 52a, ledit ergot 72 ne subit aucune force axiale par ladite came 52, de sorte que lorsque l'utilisateur fait pivoter l'organe d'actionnement 50 de sa position de repos vers sa position d'actionnement, l'ergot 72 ne bouge pas au début dudit actionnement, comme visible sur les figures 1 et 2. Avantageusement, ladite première partie 52a est plus large que ledit ergot 72. Lorsque l'ergot 72 entre en contact avec la paroi inférieure de la seconde came 52, il coopère alors avec la seconde partie 52b de ladite came, comme visible sur les figures 3 à 6. Avantageusement, la seconde partie 52b est oblique axialement, de sorte que lorsque l'utilisateur fait davantage pivoter l'organe d'actionnement 50 vers sa position d'actionnement, l'ergot 72 est poussé axialement vers le haut en coulissant dans ladite second partie 52b, ce qui déplace axialement ledit manchon d'actionnement 70 vers le haut, provoquant le déplacement axial vers le haut de la tige de piston 40, sous l'effet de la poussée exercée par le ou les crochet(s) 71 sur la denture 45. Ce déplacement axial de la tige de piston 40 déplace le piston 20 dans le réservoir 10 et génère donc la distribution d'une dose du produit fluide contenu dans le réservoir.

Un ressort de rappel 80 est monté entre l'organe d'actionnement 50 et le corps 1 pour ramener ledit organe d'actionnement 50 dans sa position de repos après chaque actionnement.

Le fonctionnement du dispositif représenté sur les figures est le suivant.

Dans la position de repos des figures 1 et 2, le réservoir 10 est isolé de l'atmosphère d'une part par le piston 20 et d'autre part par la membrane 30.

Lorsque l'utilisateur appuie sur l'organe d'actionnement 50, il va d'abord déplacer le réservoir 10 par rapport au corps 1, pour ainsi percer la membrane 30 au moyen de l'élément de perçage 4.

Une poursuite du déplacement de l'organe d'actionnement 50 va ensuite provoquer un déplacement du piston 20 à l'intérieur du réservoir 10 et donc la distribution d'une dose de produit fluide. Le produit fluide est donc poussé par ledit piston 20 à travers l'élément de perçage 4 vers le profil de pulvérisation 5, puis hors du dispositif à travers l'orifice de distribution 2.

Ainsi, c'est le dimensionnement de la denture 45 qui définit la dose de produit fluide, et il suffit de modifier cette denture sur la tige de piston 40 pour modifier le dosage, sans avoir à modifier le reste du dispositif. De même, le nombre de doses distribuables peut être aisément modifié, correspondant au nombre de dents dans la denture 45.

Avantageusement, en début d'actionnement, l'organe d'actionnement 50 coopère avec le corps 1 pour réaliser une accumulation d'énergie dans la main de l'utilisateur. Ce n'est qu'en dépassant un seuil d'effort prédéterminé que l'organe d'actionnement 50 peut être déplacé vers sa position d'actionnement, l'énergie ainsi accumulée dans les doigts de l'utilisateur permettant de générer une force suffisante pour d'abord percer la membrane 30 puis pour déplacer le piston 20 dans le réservoir 10, garantissant ainsi la distribution de la dose complète. Avantageusement, cette accumulation d'énergie peut être réalisée par une projection déformable 55 de l'organe d'actionnement 50 qui coopère avec une butée 90 du corps 1. Cette butée 90 peut être formée directement sur le corps 1 ou sur une partie fixée audit corps 1. La projection déformable 5 peut être définie par une découpe 56 qui permet la déformation de ladite projection déformable. Dans l'exemple représenté, ladite découpe 56 est sensiblement horizontale, de sorte que la déformation de la projection 55 est sensiblement axiale. D'autres mises en oeuvre sont toutefois aussi possibles pour réaliser cette accumulation d'énergie en début d'actionnement.

Il est à noter que les cames 51 et 52 peuvent être modifiées pour personnaliser la séquence d'actionnement.

Après distribution d'une dose de produit fluide, le dispositif est dans la position représentée sur la figure 6.

Lorsque l'utilisateur relâche la pression sur l'organe d'actionnement 50, le ressort 80 va le ramener vers sa position de repos.

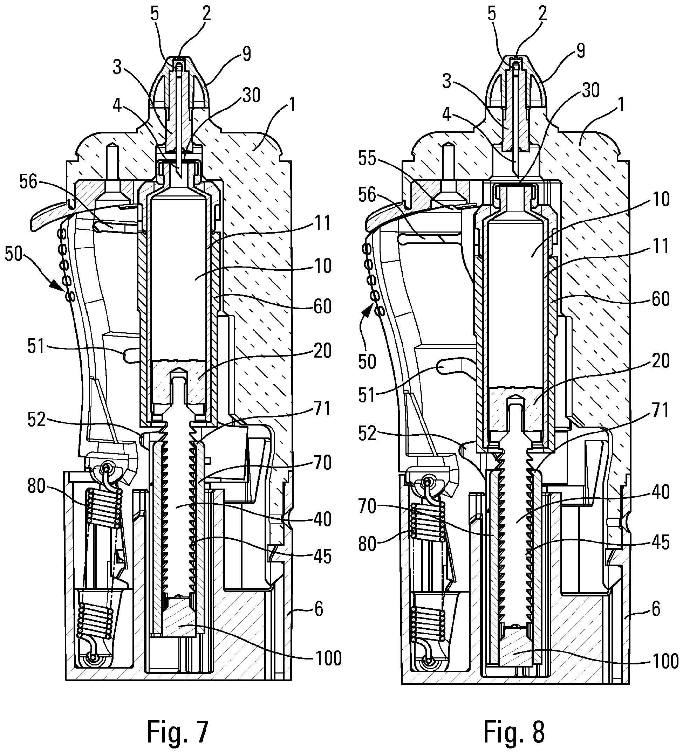

Au début de la course de retour, l'ergot 61 se déplace dans la seconde partie 51b de la première came 51, sans que cela ne génère de déplacement axial pour le réservoir 10. Par contre, l'ergot 72 va être poussé vers le bas par la seconde partie 52b de la seconde came 52, ce qui va déplacer le manchon d'actionnement 70 axialement vers le bas, avec le crochet 71 qui va s'encliqueter dans la dent suivante de la denture 45, comme visible sur la figure 7.

Avantageusement, la denture 45 de la tige de piston 40 coopère avec un cliquet anti-retour approprié, qui permet le déplacement axialement vers le haut de la tige de piston 45 et bloque son déplacement axialement vers le bas. Ainsi, lorsque l'organe d'actionnement 50 se déplace de sa position de repos vers sa position d'actionnement, le crochet 71 pousse sur une dent de la tige de piston 40 pour la déplacer axialement vers le haut, avec le cliquet anti-retour qui passe derrière la dent suivante. De manière similaire, lorsque l'organe d'actionnement 50 revient de sa position d'actionnement vers sa position de repos, le cliquet anti-retour bloque le déplacement axial vers le bas de la tige de piston 40, et le crochet 71 passe derrière la dent suivante de la denture 45.

A la fin de la course de retour, l'ergot 72 se déplace dans la première partie 52b de la seconde came 52, ce qui ne génère plus de déplacement axial pour le manchon d'actionnement 70. Par contre, l'ergot 61 va être poussé vers le bas par la seconde partie 51b de la première came 51, ce qui va déplacer le réservoir 10 axialement vers le bas, avec l'élément de perçage 4 qui va ressortir du réservoir 1 et de la membrane 30, comme visible sur la figure 8.

Lorsque l'élément de perçage 4 ressort hors du réservoir 10, la membrane 30 se referme, pour isoler à nouveau le contenu du réservoir de l'atmosphère.

En fin de course de retour, l'organe d'actionnement 50 revient en position de repos, avec la projection déformable 55 qui se déforme grâce à la découpe 56 et revient se positionner contre la butée 90.

Le dispositif est alors prêt pour l'actionnement suivant.

Ainsi, la présente invention permet, grâce à l'actionnement latéral, de réduire la dimension axiale du dispositif en déplaçant l'organe d'actionnement 50 sur le côté. Elle permet aussi de réaliser la totalité du cycle d'actionnement avec une seule action de l'utilisateur, à savoir l'appui puis le relâchement de l'organe d'actionnement 50, et donc avec une seule main.

La membrane 30 est adaptée à se refermer de manière étanche après chaque actionnement, lorsque l'élément de perçage 4 ressort du réservoir 10. Ceci garantit l'intégrité du produit fluide antre deux actionnements.

Avantageusement, la membrane 30 peut être formée dans une structure à deux couches, avec deux matières différentes :
- une couche interne, en contact avec le produit fluide, réalisée en butyle, neutre vis-à-vis du produit fluide, typiquement avec une épaisseur d'environ 0,6-0,7 mm,
- une couche externe réalisée en polyisoprène, pour la propriété de fermeture automatique, typiquement avec une épaisseur d'environ 1-1.5 mm).

Avantageusement, la membrane est réalisée par calandrage et réticulation, puis découpe, notamment par poinçonnage. Dans une première variante, les deux matières premières sont calandrées et réticulées ensemble. Dans une seconde variante, une première matière est calandrée et réticulée, puis la seconde matière est calandrée sur la première puis réticulée.

D'autres matériaux ayant des propriétés similaires sont envisageables. De même, une structure monocouche, de préférence en butyle, serait envisageable si le nombre de doses à distribuer est faible, typiquement entre 2 et 5 doses. De même, une membrane avec plus de deux couches serait également possible.

La présente invention a été décrite en référence à un mode de réalisation avantageux qui n'est pas limitatif, et toute modification utile peut être apportée à la présente invention sans sortir du cadre de celle-ci tel que défini par les revendications annexées.

## Revendications

1. Dispositif de distribution de produit fluide comportant un corps (1) pourvu d'un orifice de distribution (2) et d'un élément de perçage (4), ledit corps (1) contenant un réservoir (10) contenant au moins deux doses de produit fluide, ledit réservoir (10) comportant une ouverture axiale proximale obturée par une membrane (30) et une ouverture axiale distale obturée par un piston (20), monté coulissant dans ledit réservoir (10) pour distribuer une dose de produit fluide à chaque actionnement, ledit réservoir (10) étant axialement déplaçable par rapport audit corps (1) entre une position de repos, dans laquelle ledit élément de perçage (4) ne traverse pas ladite membrane (30), et une position de distribution, dans laquelle ledit élément de perçage (4) traverse ladite membrane (30), ladite membrane (30) étant adaptée à se refermer de manière étanche après chaque actionnement, lorsque ledit élément de perçage (4) ressort dudit réservoir (10), **caractérisé en ce que** ledit dispositif comporte un système d'actionnement latéral comprenant un organe d'actionnement (50), déplaçable latéralement par rapport audit corps (1) entre une position de repos et une position d'actionnement, ledit organe d'actionnement (50) étant élastiquement sollicité vers sa position de repos, ledit organe d'actionnement (50) comportant une première came (51) coopérant avec ledit réservoir (10) et une seconde came (52) coopérant avec ledit piston (20), de telle sorte que le déplacement dudit organe d'actionnement (50) vers sa position d'actionnement déplace d'abord ledit réservoir (10) vers sa position de distribution puis fait coulisser ledit piston (20) dans ledit réservoir (10), pour distribuer une dose de produit fluide.

2. Dispositif selon la revendication 1, dans lequel ledit organe d'actionnement (50) est monté pivotant sur une partie de corps inférieur (6), fixée audit corps (1).

3. Dispositif selon la revendication 1 ou 2, dans lequel ledit organe d'actionnement (50) est élastiquement sollicité vers sa position de repos par un ressort de rappel (80).

4. Dispositif selon l'une quelconque des revendications précédentes, dans lequel ladite première came (51) et ladite seconde came (52) sont formées par des rainures ou fentes réalisées dans ledit organe d'actionnement (50).

5. Dispositif selon la revendication 4, dans lequel ladite première came (51) coopère avec au moins un ergot (61) solidaire dudit réservoir (10), ledit ergot (61) coulissant dans ladite première came (51) lors de l'actionnement, et ladite seconde came (52) coopère avec au moins un ergot (72) solidaire dudit piston (20), ledit ergot (72) coulissant dans ladite seconde came (52) lors de l'actionnement.

6. Dispositif selon la revendication 5, dans lequel ladite première came (51) comporte une première partie (51a) oblique axialement et une seconde partie (51b) sensiblement horizontale, ladite première partie (51a) coopérant avec ledit ergot (61) au début de l'actionnement pour le pousser axialement vers le haut, ce qui déplace axialement ledit réservoir (10) vers le haut, provoquant le perçage de ladite membrane (30) par ledit élément de perçage (4), et ladite seconde partie (51b) coopère avec ledit ergot (61) en fin d'actionnement, de sorte qu'après perçage de ladite membrane (30), ledit réservoir (10) n'est pas davantage poussé vers le haut.

7. Dispositif selon la revendication 6, dans lequel ledit ergot (61) est formé sur un organe de support (60) fixé audit réservoir (10).

8. Dispositif selon l'une quelconque des revendications 5 à 7, dans lequel ladite seconde came (52) comporte une première partie (52a) et une seconde partie (52b) oblique axialement, ladite première partie (52a) coopérant avec ledit ergot (72) au début de l'actionnement de sorte que ledit ergot (72) ne subit aucune force axiale par ladite came (52) et ne bouge pas au début dudit actionnement, et ladite seconde partie (52b) coopérant avec ledit ergot (72) en fin d'actionnement pour pousser axialement vers le haut ledit ergot (72), ce qui déplace axialement vers le haut ledit piston (20) dans ledit réservoir (10) pour distribuer une dose du produit fluide contenu dans ledit réservoir (10).

9. Dispositif selon la revendication 8, dans lequel ladite première partie (52a) est plus large que ledit ergot (72).

10. Dispositif selon la revendication 8 ou 9, dans lequel ledit ergot (72) est formé sur un manchon d'actionnement (70) coopérant avec une tige de piston (40) fixée audit piston (20).

11. Dispositif selon la revendication 10, dans lequel ladite tige de piston (40) comporte une denture (45) coopérant avec au moins un crochet (71) dudit manchon d'actionnement (70).

12. Dispositif selon la revendication 10 ou 11, dans lequel un élément d'indication (100) est fixé à ladite tige de piston (40), ledit élément d'indication étant visible dans une fenêtre de visualisation (F), avantageusement pourvue d'indices d'indication tels que des graduations (G) et/ou des indices alphanumériques et/ou colorés.

13. Dispositif selon l'une quelconque des revendications précédentes, dans lequel ledit organe d'actionnement (50) comporte une projection déformable (55) coopérant en début d'actionnement avec une butée (90) du corps (1) pour générer une accumulation d'énergie dans la main de l'utilisateur.

14. Dispositif selon l'une quelconque des revendications précédentes, dans lequel ledit élément de perçage (4) est solidaire d'un insert (3) fixé dans ledit corps (1), en amont dudit orifice de distribution (2).

15. Dispositif selon l'une quelconque des revendications précédentes, dans lequel un profil de pulvérisation (5) est prévu directement en amont dudit orifice de distribution (2).

## Patentansprüche

1. Vorrichtung zur Ausgabe eines Fluidprodukts, die einen Körper (1) aufweist, der mit einer Ausgabeöffnung (2) und einem Durchstechelement (4) versehen ist, wobei der Körper (1) einen Behälter (10) enthält, der wenigstens zwei Fluidproduktdosen enthält, wobei der Behälter (10) eine proximale Axialöffnung, die von einer Membran (30) verschlossen ist, und eine distale Axialöffnung aufweist, die von einem Kolben (20) verschlossen ist, der in dem Behälter (10) gleitend gelagert ist, um bei jeder Betätigung eine Fluidproduktdosis auszugeben, wobei der Behälter (10) bezogen auf den Körper (1) zwischen einer Ruheposition, in der das Durchstechelement (4) nicht durch die Membran (30) hindurchtritt, und einer Ausgabeposition, in der das Durchstechelement (4) durch die Membran (30) hindurchtritt, axial bewegbar ist, wobei die Membran (30) geeignet ist, sich nach jeder Betätigung wieder dicht zu verschließen, wenn das Durchstechelement (4) wieder aus dem Behälter (10) austritt, **dadurch gekennzeichnet, dass** die Vorrichtung ein seitliches Betätigungssystem aufweist, das ein Betätigungsorgan (50) umfasst, das seitlich bezogen auf den Körper (1) zwischen einer Ruheposition und einer Betätigungsposition bewegbar ist, wobei das Betätigungsorgan (50) elastisch hin zu seiner Ruheposition vorgespannt ist, wobei das Betätigungsorgan (50) einen ersten Nocken (51), der mit dem Behälter (10) zusammenwirkt, und einen zweiten Nocken (52) aufweist, der mit dem Kolben (20) zusammenwirkt, so dass die Bewegung des Betätigungsorgans (50) hin zu seiner Betätigungsposition zunächst den Behälter (10) hin zu seiner Ausgabeposition bewegt, dann den Kolben (20) im Behälter (10) gleiten lässt, um eine Fluidproduktdosis auszugeben.

2. Vorrichtung nach Anspruch 1, wobei das Betätigungsorgan (50) an einem Unterkörperabschnitt (6), der am Körper (1) befestigt ist, schwenkbar gelagert ist.

3. Vorrichtung nach Anspruch 1 oder 2, wobei das Betätigungsorgan (50) durch eine Rückstellfeder (80) elastisch hin zu seiner Ruheposition vorgespannt ist.

4. Vorrichtung nach einem der vorhergehenden Ansprüche, wobei der erste Nocken (51) und der zweite Nocken (52) von Rillen oder Spalten gebildet sind, die in dem Betätigungsorgan (50) ausgebildet sind.

5. Vorrichtung nach Anspruch 4, wobei der erste Nocken (51) mit wenigstens einem Ansatz (61) zusammenwirkt, der mit dem Behälter (10) fest verbunden ist, wobei der Ansatz (61) bei der Betätigung im ersten Nocken (51) gleitet, und der zweite Nocken (52) mit wenigstens einem Ansatz (72) zusammenwirkt, der mit dem Kolben (20) fest verbunden ist, wobei der Ansatz (72) bei der Betätigung im zweiten Nocken (52) gleitet.

6. Vorrichtung nach Anspruch 5, wobei der erste Nocken (51) einen ersten axial schrägen Abschnitt (51a) und einen zweiten, im Wesentlichen horizontalen Abschnitt (51b) aufweist, wobei der erste Abschnitt (51a) zu Beginn der Betätigung mit dem Ansatz (61) zusammenwirkt, um ihn axial nach oben zu drücken, was den Behälter (10) axial nach oben bewegt, wodurch die Membran (30) vom Durchstechelement (4) durchstochen wird, und der zweite Abschnitt (51b) am Betätigungsende mit dem Ansatz (61) zusammenwirkt, so dass nach dem Durchstechen der Membran (30) der Behälter (10) nicht weiter nach oben gedrückt wird.

7. Vorrichtung nach Anspruch 6, wobei der Ansatz (61) auf einem Tragorgan (60) gebildet ist, das am Behälter (10) befestigt ist.

8. Vorrichtung nach einem der Ansprüche 5 bis 7, wobei der zweite Nocken (52) einen ersten Abschnitt (52a) und einen zweiten, axial schrägen Abschnitt (52b) aufweist, wobei der erste Abschnitt (52a) zu Beginn der Betätigung mit dem Ansatz (72) zusammenwirkt, so dass der Ansatz (72) keine axiale Kraft durch den Nocken (52) erfährt und sich zu Beginn der Betätigung nicht bewegt, und der zweite Abschnitt (52b) am Betätigungsende mit dem Ansatz (72) zusammenwirkt, um den Ansatz (72) axial nach oben zu drücken, was den Kolben (20) im Behälter (10) axial nach oben bewegt, um eine Dosis des Fluidprodukts auszugeben, das im Behälter (10) enthalten ist.

9. Vorrichtung nach Anspruch 8, wobei der erste Abschnitt (52a) breiter als der Ansatz (72) ist.

10. Vorrichtung nach Anspruch 8 oder 9, wobei der Ansatz (72) auf einer Betätigungshülse (70) gebildet ist, die mit einer Kolbenstange (40) zusammenwirkt, die am Kolben (20) befestigt ist.

11. Vorrichtung nach Anspruch 10, wobei die Kolbenstange (40) eine Zahnung (45) aufweist, die mit wenigstens einem Haken (71) der Betätigungshülse (70) zusammenwirkt.

12. Vorrichtung nach Anspruch 10 oder 11, wobei ein Angabeelement (100) an der Kolbenstange (40) befestigt ist, wobei das Angabeelement in einem Sichtfenster (F) sichtbar ist, das vorteilhafterweise mit Angabeindizes versehen ist, wie etwa Skalen (G) und/oder alphanumerischen und/oder farbigen Indizes.

13. Vorrichtung nach einem der vorhergehenden Ansprüche, wobei das Betätigungsorgan (50) einen verformbaren Vorsprung (55) aufweist, der zu Betätigungsbeginn mit einem Anschlag (90) des Körpers (1) zusammenwirkt, um eine Energieakkumulation in der Hand des Benutzers zu erzeugen.

14. Vorrichtung nach einem der vorhergehenden Ansprüche, wobei das Durchstechelement (4) fest mit einem Einsatz (3) verbunden ist, der im Körper (1) stromauf der Ausgabeöffnung (2) befestigt ist.

15. Vorrichtung nach einem der vorhergehenden Ansprüche, wobei ein Zerstäubungsprofil (5) direkt stromauf der Ausgabeöffnung (2) vorgesehen ist.

## Claims

1. A device for dispensing a fluid product comprising a body (1) having a dispensing opening (2) and a piercing element (4), said body (1) containing a reservoir (10) containing at least two doses of fluid product, said reservoir (10) having a proximal axial opening closed by a membrane (30) and a distal axial opening closed by a piston (20), mounted in a sliding manner in said reservoir (10) in order to dispense a dose of fluid product on each actuation, said reservoir (10) being axially movable with respect to said body (1) between a rest position, in which said piercing element (4) does not pass through said membrane (30), and a dispensing position, in which said piercing element (4) passes through said membrane (30), said membrane (30) being capable of reclosing in a sealed manner after each actuation, when said piercing element (4) comes back out from said reservoir (10), **characterised in that** said device comprises a lateral actuating system comprising an actuating member (50), laterally movable with respect to said body (1) between a rest position and an actuating position, said actuating member (50) being resiliently biased towards its rest position, said actuating member (50) comprising a first cam (51) cooperating with said reservoir (10) and a second cam (52) cooperating with said piston (20), such that the movement of said actuating member (50) towards its actuating position first moves said reservoir (10) towards its dispensing position and then causes said piston (20) to slide in said reservoir (10), to dispense a dose of fluid product.

2. The device according to claim 1, whrein said actuating member (50) is pivotably mounted on a lower body portion (6), fixed to said body (1).

3. The device according to claim 1 or 2, wherein said actuating member (50) is resiliently biased towards its rest position by a return spring (80).

4. The device according to any one of the preceding claims, wherein said first cam (51) and said second cam (52) are formed by grooves or slots made in said actuating member (50).

5. The device according to claim 4, wherein said first cam (51) cooperates with at least one lug (61) integral with said reservoir (10), said lug (61) sliding in said first cam (51) during the actuation, and said second cam (52) cooperates with at least one lug (72) integral with said piston (20), said lug (72) sliding in said second cam (52) during the actuation.

6. The device according to claim 5, wherein said first cam (51) comprises an axially oblique first portion (51a) and a substantially horizontal second portion (51b), said first portion (51a) cooperating with said lug (61) at the beginning of the actuation to push it axially upwards, which axially moves said reservoir (10) upwards, causing said piercing member (4) to pierce said membrane (30), and said second portion (51b) cooperating with said lug (61) at the end of the actuation, such that after piercing said membrane (30), said reservoir (10) is not pushed further upwards.

7. The device according to claim 6, wherein said lug (61) is formed on a support member (60) fixed to said reservoir (10).

8. The device according to any one of claims 5 to 7, wherein said second cam (52) comprises a first portion (52a) and a second portion (52b) that is axially oblique, said first portion (52a) cooperating with said lug (72) at the beginning of the actuation such that said lug (72) does not undergo any axial force by said cam (52) and does not move at the beginning of said actuation, and said second portion (52b) cooperating with said lug (72) at the end of the actuation so as to push said lug (72) axially upwards, which moves said piston (20) axially upwards in said reservoir (10) in order to dispense a dose of the fluid product contained in said reservoir (10).

9. The device according to claim 8, wherein said first portion (52a) is wider than said lug (72).

10. The device according to claim 8 or 9, wherein said lug (72) is formed on an actuator sleeve (70) cooperating with a piston rod (40) fixed to said piston (20).

11. The device according to claim 10, wherein said piston rod (40) comprises toothing (45) cooperating with at least one hook (71) of said actuator sleeve (70).

12. The device according to claim 10 or claim 11, wherein an indication element (100) is fixed to said piston rod (40), said indication element being visible in a viewing window (F), advantageously provided with indication indices such as graduations (G) and/or alphanumeric and/or coloured indices.

13. The device according to any one of the preceding claims, wherein said actuating member (50) comprises a deformable projection (55) cooperating, at the beginning of the actuation, with an abutment (90) of the body (1) to generate an accumulation of energy in the user's hand.

14. The device according to any one of the preceding claims, wherein said piercing element (4) is integral with an insert (3) fixed in said body (1), upstream from said dispensing opening (2).

15. The device according to any one of the preceding claims, wherein a spray profile (5) is provided directly upstream from said dispensing opening (2).
